# EUROPÄISCHE PATENTSCHRIFT

(11) **EP 1 274 677 B1**
(45) Veröffentlichungstag und Bekanntmachung des Hinweises auf die Patenterteilung: **17.01.2007**
(21) Anmeldenummer: 01931563.9
(22) Anmeldetag: 04.04.2001
(51) Int. Cl.: C07C 249/14, C07C 251/32

(54) **VERFAHREN ZUR HERSTELLUNG VON OXIMETHERN**
METHOD FOR PRODUCING OXIME ETHERS
PROCEDE DE PRODUCTION D'ETHERS D'OXIME

(30) Priorität: 11.04.2000 DE 10017724
(43) Veröffentlichungstag der Anmeldung: 15.01.2003
(73) Patentinhaber: BASF Aktiengesellschaft, 67056 Ludwigshafen (DE)
(72) Erfinder: GÖTZ, Roland, 68809 Neulussheim (DE); WOLF, Bernd, 67136 Fussgönheim (DE); RACK, Michael, 69123 Heidelberg (DE)
(74) Vertreter: Fitzner, Uwe
(86) Internationale Anmeldenummer: PCT/EP2001/003822
(87) Internationale Veröffentlichungsnummer: WO 2001/077070

(56) Entgegenhaltungen:
- WO-A-00/18726
- A.A. FADDA: "Synthesis of some new 1-Hydroxypyrazole-2-oxides and heterocyclic oximes " INDIAN JOURNAL OF CHEMISTRY, Bd. 30b, Nr. 8, 1991, Seiten 749-753, XP001013128 in der Anmeldung erwähnt

## Beschreibung

Die vorliegende Erfindung betrifft ein Verfahren zur Herstellung von Oximethern der Formel I, 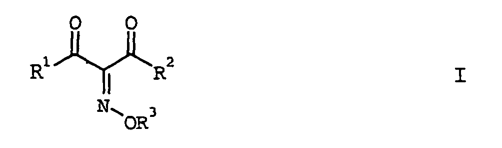 in der die Substituenten R¹ und R² gleich oder verschieden sein können und jeweils Cyano, C₁-C₄-Alkyl, C₁-C₄-Halogenalkyl, C₃-C₆-Cycloalkyl, Phenyl und Naphthyl und R³ C₁-C₄-Alkyl bedeuten können, durch Alkylierung von Oximen der Formel II, 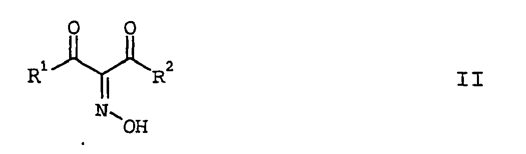 unter basischen Bedingungen mit einem Alkylierungsmittel aus der Gruppe der Alkylhalogenide, Dialkylsulfate und Dialkylcarbonate, dadurch gekennzeichnet, daß die Umsetzung in einem Gemisch, bestehend aus
5 bis 25 Gew.-% polaren aprotischen Lösungsmitteln ausgewählt aus der Gruppe Nitrile, N-Alkyl-pyrrolidone, cyclische Harnstoffderivate, Dimethylformamid und Dimethylacetamid,
55 bis 95 Gew.-% unpolaren Lösungsmitteln ausgewählt aus der Gruppe der aliphatische Kohlenwasserstoffe, aromatische Kohlenwasserstoffe, Alkylcarbonsäurealkylester und Ether und
0 bis 25 Gew.-% Wasser, wobei sich diese Anteile zu 100 % ergänzen, durchgeführt wird.

Alkylierungsverfahren für aktivierte Oxime sind aus dem Stand der Technik bekannt.

In Bull. Acad. Sci. USSR, Div. Chem. Sci. (Engl. Transl.), EN, Bd. 28, S. 121-128 (1979) werden Alkylierungsreaktionen von Oximen durch Diazoalkane, Alkylhalogenide und Dialkylsulfate beschrieben; insbesondere die Konkurrenz zwischen Sauerstoff- und Stickstoffalkylierung wird untersucht. Die Herstellung der O-Alkylether von α,α'-Bis-carbonyloximen in Aceton, Ethanol, Wasser oder Diethylether wird in mäßigen Ausbeuten beschrieben. Zur Begünstigung der 0-Alkylierung wird empfohlen, bei möglichst hohen Temperaturen zu arbeiten, da die Nitrone temperaturlabil sind, oder möglichst voluminöse Alkylierungsreagenzien zu verwenden.

In Ind. J. Chem., Bd. 30B, *S*. 749-753 (1991) wird die Methylierung der Oxime der Formel II, in der R¹ und R² für Methyl oder Phenyl stehen, beschrieben. Aus der Umsetzung mit Methyliodid in Gegenwart von wasserfreiem K₂CO₃ in Aceton werden die O-Methylether in Ausbeuten von 65, bzw. 67 % erhalten.

In WO-A 00/18726 wird die Alkylierung von Oximen der Formel II in diversen Lösungsmitteln beschrieben. Die Umsetzung kann auch in Gemischen dieser Lösungsmittel erfolgen.

Bei der Durchführung solcher Synthesen im technischen Maßstab bietet die Verwendung wasserhaltiger Lösungsmittelgemische verfahrenstechnische Vorteile. Allerdings führt ein Wasseranteil üblicherweise zu Ausbeuteverlusten.

Aufgabe der vorliegenden Erfindung war es daher, ein wirtschaftliches und großtechnisch anwendbares Verfahren zur Herstellung von O-Alkyloximethern der Formel I bereitzustellen, das auch in wasserhaltigen Lösungsmittelgemischen eine hohe Selektivität und Ausbeute aufweist und einfach durchführbar ist.

Demgemäß wurde ein Verfahren zur Herstellung von Oximethern der Formel I, 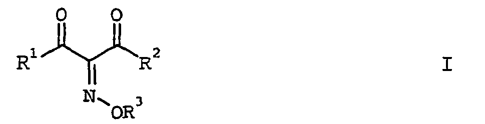 in der die Substituenten R¹ und R² gleich oder verschieden sein können und jeweils Cyano, C₁-C₄-Alkyl, C₁-C₄-Halogenalkyl, C₃-C₆-Cycloalkyl, Phenyl und Naphthyl und R³ C₁-C₄-Alkyl bedeuten können, durch Alkylierung von Oximen der Formel II, 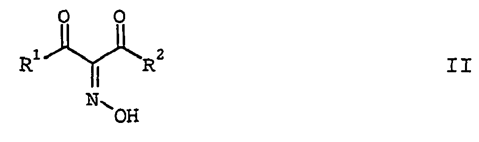 unter basischen Bedingungen mit einem Alkylierungsmittel aus der Gruppe der Alkylhalogenide, Dialkylsulfate und Dialkylcarbonate, gefunden, wobei die Umsetzung in einem Gemisch, bestehend aus
5 bis 25 Gew.-% polaren aprotischen Lösungsmitteln ausgewählt aus der Gruppe Nitrile, N-Alkyl-pyrrolidone, cyclische Harnstoffderivate, Dimethylformamid und Dimethylacetamid,
55 bis 95 Gew.-% unpolaren Lösungsmitteln ausgewählt aus der Gruppe der aliphatische Kohlenwasserstoffe, aromatische Kohlenwasserstoffe, Alkylcarbonsäurealkylester und Ether, und
ggf. bis zu 25 Gew.-% Wasser, durchgeführt wird.

In dem erfindungsgemäßen Verfahren wird entgegen der Lehre aus dem Stand der Technik bei mäßigen Temperaturen trotz Verwendung von sterisch anspruchslosen Alkylierungsmitteln und Substraten überraschenderweise keine Nitron-Bildung beobachtet und die O-Alkylether in guten bis sehr guten Ausbeuten erhalten.

Als Basen kommen allgemein salzartige Verbindungen in Betracht, wie Alkalimetall- und Erdalkalimetallcarbonate wie Natriumcarbonat, Kaliumcarbonat und Magnesiumcarbonat sowie Alkalimetallhydrogencarbonate wie Natriumhydrogencarbonat, außerdem Alkalimetallacetate wie Natriumacetat. Besonders bevorzugt wird Kaliumcarbonat.

Die Base wird im allgemeinen äquimolar oder im Überschuß verwendet; ein Überschuß von etwa 10 bis 30 % hat sich vielfach als vorteilhaft erwiesen. Die Base kann in fester Form oder als Lösung, insbesondere als wässrige Lösung, eingesetzt werden. Aus praktischen Gründen ist die Verwendung von mindestens 10 gew.-%igen bis gesättigten wässrigen Lösungen bevorzugt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, sowie N-Methylpyrrolidon, N-Octylpyrrolidon, cyclische Harnstoffderivate wie Dimethylpropylenharnstoff, Dimethylsulfoxid, Dimethylformamid und Dimethylacetamid, besonders bevorzugt werden Dimethylformamid und Dimethylacetamid, aromatische Kohlenwasserstoffe wie Toluol und Ether wie tert.-Butylmethylether.

Die Umsetzung wird in einem Lösungsmittelgemisch durchgeführt, das aus 5 bis 25 Gew.-%, bevorzugt aus 10 bis 20 Gew.-% der vorstehend genannten polaren aprotischen Lösungsmitteln, bis zu 25 Gew.-% Wasser und mindestens 5 Gew.-%, bevorzugt 55 bis 95 Gew.-% und besonders bevorzugt 60 bis 90 Gew.-%, insbesondere 60 bis 80 Gew.-% der vorstehend genannten unpolaren Lösungsmitteln besteht.

Aus verfahrenstechnischen Gründen ist die Durchführung des Verfahrens in wasserhaltigen Lösungsmittelgemischen bevorzugt.

Geeignete Alkylierungsmittel sind Alkylhalogenide, Dialkylsulfate und Dialkylcarbonate. Aus wirtschaftlichen Gründen sind Dialkylsulfate bevorzugt.

Das Alkylierungsmittel wird im allgemeinen äquimolar oder im Überschuß verwendet; ein Überschuß von etwa 5 bis 15 Gew.-% hat sich vielfach als vorteilhaft erwiesen.

Aus Sicherheitsgründen wird bei Verwendung von Alkylhalogeniden und Dialkylsulfaten nach der Reaktion üblicherweise überschüssiges Alkylierungsmittel vernichtet, meist werden dazu Ammoniak oder Amine wie z.B. Diethylamin verwendet.

In einer bevorzugten Ausführungsform des Verfahrens werden Methylierungsmittel verwendet.

Die Reaktion wird üblicherweise bei Temperaturen von 0 bis 100°C, bevorzugt bei 0 bis 50°C und ganz besonders bevorzugt bei 10 bis 25°C durchgeführt. Die Reaktionstemperatur wird üblicherweise entsprechend der Temperaturstabilität des Oxims eingestellt.

Die Reihenfolge der Zugabe der Reaktanden ist nicht kritisch. In einer bevorzugten Ausführungsform des Verfahrens wird die Base, fest oder als wässrige Lösung, in dem Lösungsmittel vorgelegt, das Oxim und das Alkylierungsmittel gleichzeitig und äquimolar dem gegebenenfalls wasserhaltigen Lösungsmittelgemisch zugegeben. In einer anderen bevorzugten Ausführungsform wird das Alkylierungsmittel teilweise oder ganz vorgelegt und das Oxim anschließend zugegeben.

Das erfindungsgemäße Verfahren ist nicht auf bestimmt substituierte Verbindungen beschränkt, sofern die Substituenten unter den Reaktionsbedingungen inert sind. Aliphatische Reste können geradkettig oder verzweigt sein. Die Kettenlänge der Substituenten ist für das erfindungsgemäße Verfahren ohne Belang, jedoch wird man aus technischen Gründen üblicherweise Reste mit höchstens 4 C-Atomen wählen.

Ganz besonders bevorzugt wird das Verfahren zur Herstellung von Pentan-2,3,4-trion-3-O-methyloxim verwendet.

Alkyl steht generell für C₁-C₁₀-Alkyl, insbesondere für C₁-C₄-Alkyl, das gilt auch für Halogenalkyl; Cycloalkylreste haben drei bis sechs Ringglieder.

Die Substituenten R¹ und R² können weitere, unter den Reaktionsbedingungen inerte Reste tragen, dabei seien beispielhaft genannt: Halogen, Cyano, SO₃H, COOH, COOR⁴, C₁-C₁₀-Alkyl, C₂-C₈-Alkenyl, C₂-C₈-Alkinyl, Phenyl oder Heteroaryl; R⁴ bedeutet C₁-C₁₀-Alkyl.

Heteroaryl bedeutet beispielsweise Furyl, Thienyl, Pyrrolyl, Isoxazolyl, Isothiazolyl, Pyrazolyl, Oxazolyl, Imidazolyl, Pyridyl, Pyridazinyl, Pyrimidinyl oder Triazinyl;

Halogen bedeutet Chlor, Fluor, Brom oder Jod.
Alkenyl- und Alkinylreste haben zwei bis acht, insbesondere drei bis sechs C-Atome.

Die nach dem erfindungsgemäßen Verfahren erhältlichen O-Alkyloximether eignen sich als Zwischenprodukte zur Herstellung von Farbstoffen oder Wirkstoffen auf dem Pharma- oder Pflanzenschutzbereich.

Die nachfolgenden Vergleichsbeispiele verdeutlichen den Einfluß der erfindungswesentlichen Merkmale; die Beispiele sollen das erfindungsgemäße Verfahren weiter erläutern:

### Vergleichsbeispiele:

### 1. Methylierung in Aceton [Ind. J. Chem., Bd. 30B, S. 749-753 (1991)]

Zu einer Suspension von 16,6 g Kaliumcarbonat in 40 ml Aceton wurden bei etwa 20°C unter Kühlung eine Lösung von 12,9 g Pentan-2,3,4-trion-3-oxim in 38,5 ml Aceton und 15,6 g Methyliodid zugetropft. Nach 2 Std. Rühren bei etwa 20°C wurde das Lösungsmittel abdestilliert. Der Rückstand wurde nach Lösen in Wasser mit tert.-Butylmethylether extrahiert. Aus den vereinigten organischen Phasen erhielt man 12 g Pentan-2,3,4-trion-3-O-methyloxim (nach GC-Analyse 97 %ig), was einer Ausbeute von 81,5 % der Theorie entspricht.

### 2. Methylierung in Dimethylformamid (DMF)

Zu einer Suspension von 16,6 g Kaliumcarbonat in 40 g DMF wurden bei etwa 20°C unter Kühlung eine Lösung von 12,9 g Pentan-2,3,4-trion-3-oxim in 38,5 g DMF und 13,9 g Dimethylsulfat (DMS) zugetropft. Nach 2 Std. Rühren bei etwa 20°C wurden 2,2 g Diethylamin zugegeben, um überschüssiges DMS zu vernichten, nach einer weiteren Stunde Rühren bei etwa 20°C wurden 130 ml Wasser zugegeben. Die Lösung wurde mit tert.-Butylmethylether extrahiert. Aus den vereinigten organischen Phasen erhielt man nach Waschen mit 1 n H₂SO₄ 12,8 g Pentan-2,3,4-trion-3-O-methyloxim (nach GC-Analyse 90 %ig), was einer Ausbeute von 80,5 % der Theorie entspricht.

### 3. Methylierung in tert.-Butylmethylether (MTBE)

Zu einer Suspension von 16,6 g Kaliumcarbonat in 40 g MTBE wurden bei etwa 20°C unter Kühlung eine Lösung von 12,9 g Pentan-2,3,4-trion-3-oxim in 38,5 g MTBE und 13,9 g Dimethylsulfat (DMS) zugetropft. Nach 2 Std. Rühren bei etwa 20°C wurden 2,2 g Diethylamin zugegeben, um überschüssiges DMS zu vernichten, nach einer weiteren Stunde Rühren bei etwa 20°C wurden 80 ml Wasser zugegeben. Die Lösung wurde mit tert.-Butylmethylether extrahiert. Aus den vereinigten organischen Phasen erhielt man nach Waschen mit 1 n H₂SO₄ 9,9 g Pentan-2,3,4-trion-3-O-methyloxim (nach GC-Analyse 76,7 %ig), was einer Ausbeute von 53 % der Theorie entspricht.

### 4. Methylierung in tert.-Butylmethylether-Aceton 87:13

Zu einer Suspension von 16,6 g Kaliumcarbonat in einem Gemisch aus 30 g tert.-Butylmethylether (MTBE) und 10 g Aceton wurden bei etwa 20°C unter Kühlung eine Lösung von 12,9 g Pentan-2,3,4-tri-on-3-oxim in 38,5 g MTBE und 13,9 g Dimethylsulfat (DMS) zugetropft. Nach 2 Std. Rühren bei etwa 20°C wurden 2,2 g Diethylamin zugegeben, um überschüssiges DMS zu vernichten, nach einer weiteren Stunde Rühren bei etwa 20°C wurden 80 ml Wasser zugegeben. Die Lösung wurde mit tert.-Butylmethylether extrahiert. Aus den vereinigten organischen Phasen erhielt man nach Waschen mit 1 n H₂SO₄ 11 g Pentan-2,3,4-trion-3-O-methyloxim (nach GC-Analyse 85,8 %ig), was einer Ausbeute von 70,0 % der Theorie entspricht.

### 5. Methylierung in DMF-MTBE 89:11

Zu einer Suspension von 16,6 g Kaliumcarbonat in einem Gemisch aus 32 g Dimethylformamid (DMF) und 8 g tert.-Butylmethylether (MTBE) wurden bei etwa 20°C unter Kühlung eine Lösung von 12,9 g Pentan-2,3,4-trion-3-oxim in 38,5 g DMF und 13,9 g Dimethylsulfat (DMS) zugetropft. Nach 2 Std. Rühren bei etwa 20°C wurden 2,2 g Diethylamin zugegeben, um überschüssiges DMS zu vernichten, nach einer weiteren Stunde Rühren bei etwa 20°C wurden 130 ml Wasser zugegeben. Die Lösung wurde mit tert.-Butylmethylether extrahiert. Aus den vereinigten organischen Phasen erhielt man nach Waschen mit 1 n H₂SO₄ 13,5 g Pentan-2,3,4-trion-3-O-methyloxim (nach GC-Analyse 91,9 %ig), was einer Ausbeute von 86,7 % der Theorie entspricht.

### 6. Methylierung in DMF-MTBE 48:52 [WO-A 00/18726]

In einem 20 1 Gefäß wurden 4,5 kg (32,6 mol) Kaliumcarbonat in 3,2 1 Methyl-tert.-butylether und einem Liter DMF suspendiert. Das Gemisch wurde unter Rühren auf 0 bis -10°C abgekühlt. Anschließend wurde eine Lösung aus 4128 g (32 mol) Pentan-2,3,4-trion-3-oxim, 2 1 DMF und 4032 g (32 mol) Diemethylsulfat innerhalb von 2 Stunden bei einer Innentemperatur von <25°C zudosiert. Es wurde 3,5 Stunden bei Raumtemperatur nachgerührt. Dann wurden 20 1 Wasser zudosiert, die obere organische Phase abgetrennt, die wäßrige Phase mit 2 1 Methyl-tert.-butylether gewaschen, die vereinigten organischen Phasen mit 1 1 5%iger Salzsäure gewaschen und das Lösungsmittel abdestilliert. Man erhielt 4214 g der Titelverbindung in einer Reinheit von 96,6 % (GC-Flächenprozent) was einer Ausbeute von 89% entspricht.

### Erfindungsgemäße Beispiele:

### 7. Methylierung in DMF-MTBE 13:87

Zu einer Suspension von 165 g Kaliumcarbonat in einem Gemisch aus 100 g Dimethylformamid (DMF) und 300 g tert.-Butylmethylether (MTBE) wurden bei etwa 20°C unter Kühlung eine Lösung von 129,0 g Pentan-2,3,4-trion-3-oxim in 385 g MTBE und 138,7 g Dimethylsulfat (DMS) zugetropft. Nach 2 Std. Rühren bei etwa 20°C wurden 21,9 g Diethylamin zugegeben, um überschüssiges DMS zu vernichten, nach einer weiteren Stunde Rühren bei etwa 20°C wurden 800 ml Wasser zugegeben. Die Lösung wurde mit tert.-Butylmethylether extrahiert. Aus den vereinigten organischen Phasen erhielt man nach Waschen mit 1 n H₂SO₄ 144,6 g Pentan-2,3,4-trion-3-O-methyloxim (nach GC-Analyse 96,5 %ig), was einer Ausbeute von 97,6 % der Theorie entspricht.

### 8. Methylierung in DMF-MTBE-Wasser 13:66:20

Zu 332 g einer 50 Gew.-%igen wässrigen Lösung von Kaliumcarbonat, 110 g Dimethylformamid (DMF), 300 g tert.-Butylmethylether (MTBE) und 7 g Dimethylsulfat (DMS) wurden bei etwa 17-19°C unter Kühlung eine Lösung von 129 g Pentan-2,3,4-trion-3-oxim in 246 g MTBE und 132 g Dimethylsulfat (DMS) zugetropft. Nach 2 Std. Rühren bei etwa 20°C wurden 36,5 g Diethylamin zugegeben, um überschüssiges DMS zu vernichten, nach einer weiteren Stunde Rühren bei etwa 20°C wurden 600 ml Wasser zugegeben. Die Lösung wurde mit tert.-Butylmethylether extrahiert. Aus den vereinigten organischen Phasen erhielt man nach Waschen mit 1 n H₂SO₄ 136,9 g Pentan-2,3,4-tri-on-3-O-methyloxim (nach GC-Analyse 96,3 %ig), was einer Ausbeute von 92,1 % der Theorie entspricht.

## Patentansprüche

1. Verfahren zur Herstellung von Oximethern der Formel I, 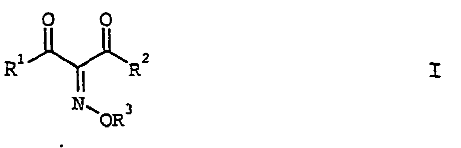 in der die Substituenten R¹ und R² gleich oder verschieden sein können und jeweils Cyano, C₁-C₄-Alkyl, C₁-C₄-Halogenalkyl, C₃-C₆-Cycloalkyl, Phenyl und Naphthyl und R³ C₁-C₄-Alkyl bedeuten können,
durch Alkylierung von Oximen der Formel II, 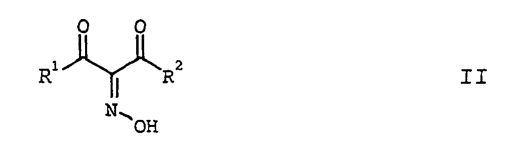 unter basischen Bedingungen mit einem Alkylierungsmittel aus der Gruppe der Alkylhalogenide, Dialkylsulfate und Dialkylcarbonate,
**dadurch gekennzeichnet, daß** die Umsetzung in einem Gemisch bestehend aus
5 bis 25 Gew.-% polaren aprotischen Lösungsmitteln ausgewählt aus der Gruppe Nitrile, N-Alkyl-pyrrolidone, cyclische Harnstoffderivate, Dimethylformamid und Dimethylacetamid,
55 bis 95 Gew.-% unpolaren Lösungsmitteln ausgewählt aus der Gruppe der aliphatische Kohlenwasserstoffe, aromatische Kohlenwasserstoffe, Alkylcarbonsäurealkylester und Ether, und
0 bis 25 Gew.-% Wasser,
wobei sich diese Anteile zu 100 % ergänzen, durchgeführt wird.

2. Verfahren nach Anspruch 1, wobei als unpolare Lösungsmittel Ether verwendet werden.

3. Verfahren nach Anspruch 2, wobei tert.-Butylmethylether verwendet wird.

4. Verfahren nach einem der Ansprüche 1 bis 3, wobei die Umsetzung in der Gegenwart von Wasser durchgeführt wird.

5. Verfahren nach einem der Ansprüche 1 bis 4, wobei als polare Lösungsmittel Dimethylformamid oder Dimethylacetamid verwendet werden.

6. Verfahren nach einem der Ansprüche 1 bis 5, wobei als Alkylierungsmittel Dialkylsulfate verwendet werden.

7. Verfahren nach einem der Ansprüche 1 bis 6, wobei der Anteil der polaren Lösungsmittel 10 bis 25 Gew.% beträgt.

8. Verfahren nach einem der Ansprüche 1 bis 7, wobei Basen aus der Gruppe der Hydrogencarbonate, Carbonate und Acetate verwendet werden.

9. Verfahren nach Anspruch 8, wobei Alkalimetall- oder Erdalkalimetallcarbonate, Alkalimetallhydrogencarbonate oder Alkalimetallacetate verwendet werden.

10. Verfahren nach Anspruch 8, wobei Kaliumcarbonat verwendet wird.

11. Verfahren nach einem der Ansprüche 1 bis 10, wobei in Formel I die Substituenten R¹, R² und R³ C₁-C₄-Alkyl bedeuten.

## Claims

1. Process for preparing oxime ethers of the formula I, 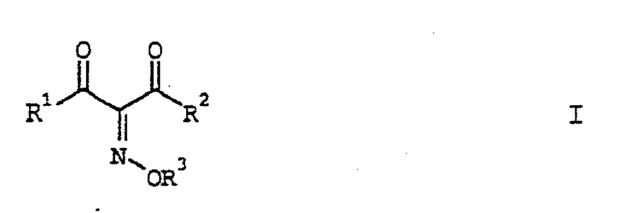 in which the substituents R¹ and R² can be identical or different and can each be cyano, C₁-C₄-alkyl, C₁-C₄-haloalkyl, C₃-C₆-cycloalkyl, phenyl and naphthyl, and R³ can be C₁-C₄-alkyl,
by alkylation of oximes of the formula II 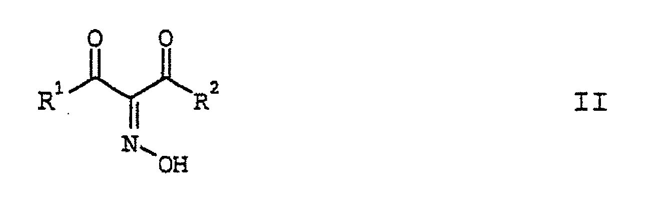 under basic conditions with an alkylating agent from the group of alkyl halides, dialkyl sulphates and dialkyl carbonates,
**characterized in that** the reaction is carried out in a mixture consisting of
5 to 25% by weight of polar aprotic solvents selected from the group of nitriles, N-alkyl-pyrrolidones, cyclic urea derivatives, dimethylformamide and dimethylacetamide,
55 to 95% by weight of nonpolar solvents selected from the group of aliphatic hydrocarbons, aromatic hydrocarbons, alkyl alkylcarboxylates and ethers, and
0 to 25% by weight of water,
the contents thereof totalling 100%.

2. Process according to Claim 1, where ethers are used as nonpolar solvents.

3. Process according to Claim 2, where tert-butyl methyl ether is used.

4. Process according to any of Claims 1 to 3, where the reaction is carried out in the presence of water.

5. Process according to any of Claims 1 to 4, where dimethylformamide or dimethylacetamide are used as polar solvents.

6. Process according to any of Claims 1 to 5, where dialkyl sulphates are used as alkylating agents.

7. Process according to any of Claims 1 to 6, where the content of polar solvents is 10 to 25% by weight.

8. Process according to any of Claims 1 to 7, where bases from the group of bicarbonates, carbonates and acetates are used.

9. Process according to Claim 8, where alkali metal or alkaline earth metal carbonates, alkali metal bicarbonates or alkali metal acetates are used.

10. Process according to Claim 8, where potassium carbonate is used.

11. Process according to any of Claims 1 to 10, where the substituents R¹, R² and R³ in formula I are each C₁-C₄-alkyl.

## Revendications

1. Procédé de préparation d'éthers d'oxime de la formule I : 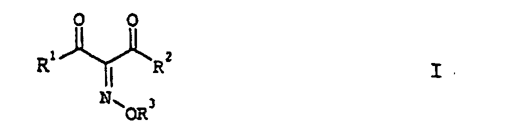 dans laquelle les substituants R¹ et R² peuvent être identiques ou différents et représenter chacun un groupe cyano, alkyle en C₁-C₄, halogénoalkyle en C₁-C₄, cycloalkyle en C₃-C₆, phényle ou naphtyle et R³ peut représenter un groupe alkyle en C₁-C₄,
par alkylation d'oximes de la formule II : 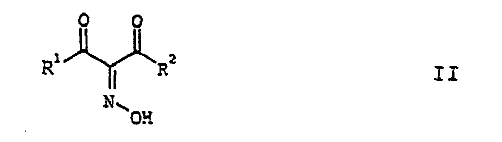 dans des conditions basiques avec un agent d'alkylation du groupe des halogénures d'alkyle, des sulfates de dialkyle et des carbonates de dialkyle,
**caractérisé en ce que** la réaction est effectuée dans un mélange constitué
de 5 à 25 % en poids de solvants aprotiques polaires choisis parmi le groupe des nitriles, des N-alkyl-pyrrolidones, des dérivés d'urée cycliques, du diméthylformamide et du diméthylacétamide,
de 55 à 95 % en poids de solvants non polaires choisis parmi le groupe des hydrocarbures aliphatiques, des hydrocarbures aromatiques, des alkylcarboxylates d'alkyle et des éthers, et
de 0 à 25 % en poids d'eau,
ces fractions se complétant pour donner 100 %.

2. Procédé suivant la revendication 1, dans lequel on utilise des éthers comme solvants non polaires.

3. Procédé suivant la revendication 2, dans lequel on utilise de l'éther tert-butylméthylique.

4. Procédé suivant l'une des revendications 1 à 3, dans lequel la réaction est effectuée en présence d'eau.

5. Procédé suivant l'une des revendications 1 à 4, dans lequel on utilise, comme solvants polaires, du diméthylformamide ou du diméthylacétamide.

6. Procédé suivant l'une des revendications 1 à 5, dans lequel on utilise, comme agents d'alkylation, des sulfates de dialkyle.

7. Procédé suivant l'une des revendications 1 à 6, dans lequel la fraction des solvants polaires est de 10 à 25 % en poids.

8. Procédé suivant l'une des revendications 1 à 7, dans lequel on utilise des bases du groupe des bicarbonates, des carbonates et des acétates.

9. Procédé suivant la revendication 8, dans lequel on utilise des carbonates de métal alcalin ou de métal alcalino-terreux, des bicarbonates de métal alcalin ou des acétates de métal alcalin.

10. Procédé suivant la revendication 8, dans lequel on utilise du carbonate de potassium.

11. Procédé suivant l'une des revendications 1 à 10, dans lequel, dans la formule I, les substituants R¹, R² et R³ représentent un groupe alkyle en C₁-C₄.
